**Europäisches Patentamt**

**European Patent Office**

**Office européen des brevets**

(19)

(11) Veröffentlichungsnummer: **0 240 815 B1**

# EUROPÄISCHE PATENTSCHRIFT

(12)

(45) Veröffentlichungstag der Patentschrift:
20.02.91 Patentblatt 91/08

(51) Int. Cl.$^5$: **A61F 2/36**

(21) Anmeldenummer: **87104196.8**

(22) Anmeldetag: **21.03.87**

(54) **Blattartiger Schaft für die Verankerung einer Hüftgelenksprothese im Femur.**

(30) Priorität: **03.04.86 CH 1302/86**

(43) Veröffentlichungstag der Anmeldung:
**14.10.87 Patentblatt 87/42**

(45) Bekanntmachung des Hinweises auf die
Patenterteilung:
**20.02.91 Patentblatt 91/08**

(84) Benannte Vertragsstaaten:
**AT BE DE ES FR GB IT NL SE**

(56) Entgegenhaltungen:
EP-A- 0 027 159
MEDICAL PROGRESS TECHNOLOGY, Band 5,
Nr. 2, September 1977, Seiten 73-102, Springer
Verlag, DE; P.J. BROCKHURST et al.: "Design
of total hip prosthesis"

(73) Patentinhaber: **GEBRÜDER SULZER
AKTIENGESELLSCHAFT
Zürcherstrasse 9
CH-8401 Winterthur (CH)**

(72) Erfinder: **Deckner, André Georges
5, Rue de l'Harmonie
F-75015 Paris (FR)**
Erfinder: **Imhof, Martin
Schöngrund 10
CH-6343 Rotkreuz (CH)**
Erfinder: **Zweymüller, Karl, Prof. Dr.-med.
Orthopädische Universitätsklinik Garnisonstr.
13
A-1090 Wien (AT)**

(74) Vertreter: **Sparing Röhl Henseler
Patentanwälte European Patent Attorneys
Rethelstrasse 123
D-4000 Düsseldorf 1 (DE)**

## Beschreibung

Die Erfindung betrifft einen blattartigen Schaft für die Verankerung einer Hüftgelenkprothese im Femur, welcher Schaft mit einem Prothesenhals verbunden ist und sich vom distalen Ende in Richtung seiner Längsachse allseitig konisch erweitert, wobei seine mediale Schmalseite aus dem Konus heraus in einen stetig gekrümmten Bogen übergeht, der am Schnittpunkt der den distalen Rand des Prothesenhalses enthaltenden Prothesenhals-Abschlussebene mit der medialen Schaftkontür endet, und wobei ferner die laterale Schmalseite aus der konischen Erweiterung heraus zu einem Trochanterflügel ausgeweitet ist, ehe sie über eine Schulter des Schaftblattes in die Prothesenhals-Abschlussebene einmündet.

Schäfte der vorstehend genannten Art sind bekannt ; beispielsweise verweisen wir dazu auf die CH-A-642 252 oder CH-A-650 407. Bei allen bisherigen Konstruktionen dieser Art ist die mediale Schmalseite in einem Kreisbogen zur Prothesenhals-Abschlussebene geführt.

Wird eine Verankerung derartiger Schäfte im Diaphysen-Bereich, d.h. in seinem distalen Teil, durchgeführt, so ist es notwendig, dass sich der Schaft einige Zeit nach der Implantation "setzen" kann, damit er sich durch ein tieferes Einsinken in den Femur fest verkeilt. Bei Schäften mit im Kreisbogen geführter medialer Begrenzung besteht die Gefahr, dass sie am distalen Ende des Calcar-Bogens an der Kortikalis anstehen, so dass ein Einsinken verhindert wird.

Aufgabe der Erfindung ist es, die mediale Begrenzung derartiger Schäfte so zu gestalten, dass dieser Nachteil vermieden wird ; mit der Erfindung wird diese Aufgabe dadurch gelöst, dass der Bogen der medialen Schmalseite Teil eines Hyperbelastes ist, dessen Scheitelpunkt im Schnittpunkt der Prothesenhals-Abschlussebene mit der medialen Schaftkontur liegt, wobei die Hyperbel nach distal in den Konus in einem gegebenen Punkt einmündet, in dem ihre Tangente mit der Schaftachse einen Winkel gleich dem halben Konuswinkel bildet.

Durch die Hyperbelform wird eine verbesserte Anpassung des medialen Schaftbereichs an den Verlauf des Calcar-Bogens erreicht. Vor allem wird dadurch ein Anstossen des Schaftes am distalen Ende des Calcar-Bogens vermieden.

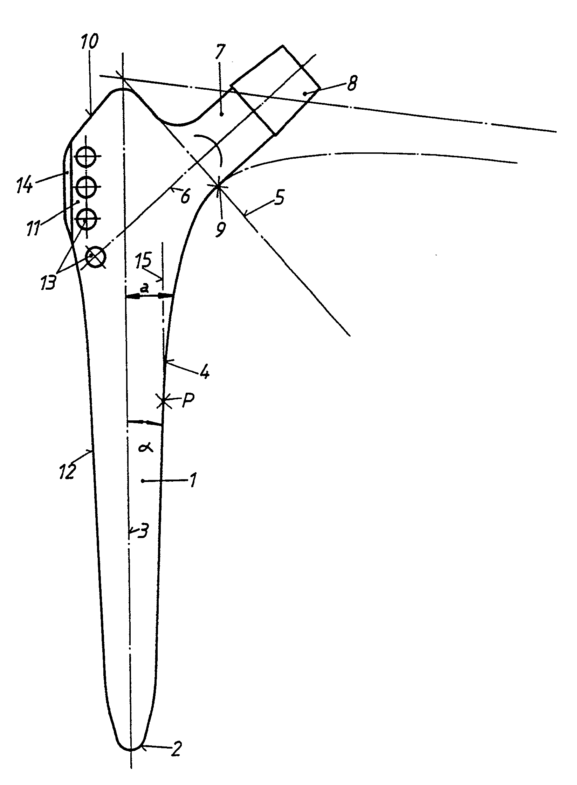

Im folgenden wird die Erfindung anhand eines Ausführungsbeispiels im Zusammenhang mit der Zeichnung näher erläutert.

Die einzige Figur zeigt schematisch eine Ausführungsform des neuen Schaftes in einer Ansicht anterior/posterior.

Das Schaftblatt 1 des Prothesenschaftes erweitert sich von einem distalen Ende 2 nach allen Seiten konisch, wobei der Konus symmetrisch zu einer Längsmittelachse ausgebildet ist. Die mediale Schmalseite 4 des Konus geht in einen Bogen über, der in einer Ebene 5 endet, die senkrecht zur Prothesenhalsachse 6 verlaufend den Prothesenhals 7 gegen das Schaftblatt 1 abschliesst.

Der Prothesenhals 7 endet in einem sich nach aussen konisch verjüngenden Zapfen 8, auf den ein nicht gezeigter kugelförmiger Gelenkkopf aufsteckbar ist.

Erfindungsgemäss ist der Bogen der medialen Schmalseite 4 eine Hyperbel, deren Scheitelpunkt im Schnittpunkt 9 des Bogens mit der Ebene 5 liegt. Weiterhin geht die Hyperbel in einem Punkt P in den distalen Konus über, in dem ihre Tangente 15 mit der Längsachse 3 des Schaftes einen Winkel $\alpha$ bildet, der gleich dem halben Konuswinkel des distalen Konus ist.

Durch die Hyperbelform ergibt sich, dass die Strecke a zwischen der Längsachse 3 und der medialen Schmalseite 4 in Richtung nach distal aus dem Bogen der Schmalseite 4 heraus langsamer abnimmt als bei einem Kreisbogen. Auf diese Weise wird eine verbesserte Anpassung der Form des Schaftes an die anatomischen Gegebenheiten im Bereich des Calcar-Bogens erreicht.

Lateral vom Prothesenhals 7 bildet die Ebene 5 einen Teil eines dachfirstartig ausgebildeten proximalen Randes 10 des Schaftblattes, der nach distal in einen Trochanterflügel 11 übergeht. Von diesem führt, sich konisch verjügend, die laterale Schmalseite 12 zum distalen Ende 2 des Schaftblattes 1.

Im Trochanterflügel 11 sind Durchbrüche 13 vorgesehen, die für eine Identifikation der Prothese und/ oder für eine Beobachtung der Knochenstruktur im Röntgenbild dienen. Weiterhin weist das äussere Ende des Trochanterflügels 11 eine Verjüngung 14 auf.

## Ansprüche

1. Blattartiger Schaft für die Verankerung einer Hüftgelenkprothese im Femur, welcher Schaft mit einem Prothesenhals (7) verbunden ist und sich vom distalen Ende (2) in Richtung seiner Längsachse (3) allseitig konisch erweitert, wobei seine mediale Schmalseite (4) aus dem Konus heraus in einen stetig gekrümmten Bogen übergeht, der am Schnittpunkt (9) der den distalen Rand des Prothesenhalses (7) enthaltenden Prothesenhals-Abschlussebene (5) mit der medialen Schaftkontur endet, und wobei ferner die laterale Schmalseite (12) aus der konischen Erweiterung heraus zu einem Trochanterflügel (11) ausgeweitet ist, ehe sie über eine Schulter des Schaftblattes (1) in die Prothesenhals-Abschlussebene (5) einmündet, dadurch gekennzeichnet, dass der Bogen der medialen Schmalseite Teil eines Hyperbelastes ist, dessen Scheitelpunkt im Schnittpunkt (9) der Prothesenhals-Abschlussebene (5) mit der medialen Schaftkontur liegt, wobei die Hyperbel nach distal in den Konus in einem gegebenen Punkt (P) einmündet, in dem ihre Tangente mit der Schaftachse (3) einen Winkel (α) gleich dem halben Konuswinkel bildet.

## Claims

1. A blade-like stem for anchoring a hip joint replacement in the femur, the stem (1) being connected to a prosthesis neck (7) and widening conically in all directions from the distal end (2) along its longitudinal axis (3), its medial narrow side (4) merging out of the cone into a continuous curve ending at the intersection (9) with the medial stem contour of the neck termination plane (5) which contains the distal edge of the neck (7), the lateral narrow side (12) widening out from the conical widening to a trocanter wing (11) before entering by way of a shoulder of the stem blade (1) the neck termination plane (5), characterised in that the curve of the medial narrow side is part of a hyperbola branch whose apex is disposed at the intersection (9) of the neck termination plane (5) with the medial shaft contour, the hyperbola extending in the distal direction into the cone at a given place (P) where its tangent forms with the stem axis (3) an angle (α) equal to half the cone angle.

## Revendications

1. Tige en forme de feuille pour l'ancrage d'une prothèse coxofémorale dans le fémur, ladite tige (1) étant reliée à un col prothétique (7) et s'évasant en cône de toutes parts dans la direction de son axe longitudinal (3), à partir de l'extrémité distale (2), son petit côté interne (4) se prolongeant, à partir du cône, par un arc à courbure continue qui s'achève au point d'intersection (9) du contour interne de la tige et du plan de terminaison (5) du col prothétique renfermant le bord distal du col prothétique (7), et le petit côté latéral (12) s'évasant par ailleurs en une aile trochantérienne (11), à partir de l'évasement conique, avant de déboucher dans le plan de terminaison (5) du col prothétique par un épaulement de la feuille (1) de la tige, caractérisée par le fait que l'arc du petit côté interne est une partie d'une branche d'hyperbole dont le sommet se trouve au point d'intersection (9) du contour interne de la tige et du plan de terminaison (5) du col prothétique, l'hyperbole aboutissant au cône, vers la région distale, en un point donné (P) auquel sa tangente forme, avec l'axe (3) de la tige, un angle (α) égal à la moitié de l'angle du cône.